# EUROPEAN PATENT APPLICATION

(11) **EP 0 879 614 A1**
(43) Date of publication of application: **25.11.1998**
(21) Application number: 98304007.2
(22) Date of filing: 20.05.1998
(51) Int. Cl.: A61M 25/01, A61N 5/10

(54) **Catheter and guide wire assembly for delivery of a radiation source**

(30) Priority: 20.05.1997 US 859217
(71) Applicant: Advanced Cardiovascular Systems, Inc., Santa Clara, California 95054 (US)
(72) Inventor: Nagy, John S., Marina del Rey, California 90292 (US)
(74) Representative: McLeish, Nicholas Alistair Maxwell

(57) **Abstract**

A guide wire (25, 80) for use in intravascular procedures having a proximal hollow portion (26, 92) and a solid distal portion (30). The hollow portion (26, 92) is adapted to receive a radioactive source material (51) for irradiating a body lumen (34) to reduce the likelihood of the development of restenosis.

## Description

### BACKGROUND OF THE INVENTION

### Field of the Invention

The present invention relates to an apparatus and method for reducing the likelihood of development of restenosis after an arterial intervention and, more particularly, to a hollow guide wire for delivery of a radioactive source to the site of the lesion following, or during, an intravascular procedure such as percutaneous transluminal coronary angioplasty (PTCA).

### Description of the Prior Art

In PTCA procedures, a guiding catheter having a pre-shaped distal tip is introduced percutaneously into the cardiovascular system of a patient and advanced therein until the pre-shaped distal tip is disposed within the aorta, adjacent to the ostium of the desired coronary artery. The guiding catheter is twisted or torqued from the proximal end to turn the distal tip of the guiding catheter so that it can be guided into the coronary ostium. In an over-the-wire dilatation catheter system, a dilatation catheter having a balloon on its distal end and a guide wire slidably disposed within an inner lumen of the dilatation catheter are introduced into and advanced through the guiding catheter to its distal tip. The distal tip of the guide wire usually is manually shaped (i.e., curved) before the guide wire is introduced into the guiding catheter along with the dilatation catheter. The guide wire usually is first advanced out the distal tip of the guiding catheter, into the patient's coronary artery, and torque is applied to the proximal end of the guide wire, which extends out of the patient. to guide the curved or otherwise shaped distal end of the guide wire as the guide wire is advanced within the coronary anatomy until the distal end of the guide wire enters the desired artery. The advancement of the guide wire within the selected artery continues until its distal end crosses the lesion to be dilated. The dilatation catheter then is advanced out of the distal tip of the guiding catheter, over the previously advanced guide wire, until the balloon on the distal extremity of the dilatation catheter is properly positioned across the lesion. Once properly positioned. the dilatation balloon is inflated to a pre-determined size (preferably the same as the normal inner diameter of the artery at that particular location) with radiopaque liquid at relatively high pressures (e.g., 4.052 to 12.16 bars (4-12 atmospheres)) to dilate the stenosed region of the diseased artery. The balloon then is deflated so that the dilatation catheter can be removed from the dilated stenosis and blood flow can resume through the dilated artery.

A rapid-exchange catheter has a relatively short guide wire-receiving sleeve or inner lumen (sometimes referred to as the "rail") which extends a short distance through the distal portion of the catheter body. This inner lumen preferably extends approximately 10 cm. and typically about 30 to 40 cm. from a first guide wire port at the distal end of the catheter to a second side guide wire port located on the catheter body. In some catheters the rail can be much smaller than 10 cm. especially when the side guide wire port is located distal to the inflation balloon. The catheter can be advanced within the patient's vascular system in much the same fashion as described above, as the short, guide wire lumen of the catheter slides along the length of the guide wire. Alternatively, the guide wire first may be advanced within the patient's vasculature until the distal end of the guide wire extends distally to the stenosis with the catheter then being mounted onto the proximal end of the in-place guide wire and advanced over the guide wire until the balloon portion is positioned across the stenosis. This particular structure allows for the rapid-exchange of the catheter, usually without the need for an exchange wire or adding a guide wire extension to the proximal end of the guide wire. Other over-the-wire or rapid-exchange catheters also can be designed to utilize therapeutic or diagnostic means in place of the balloon in the description above.

Several notable improvements recently have been made in balloon angioplasty catheters. One such improvement is described in U.S. Pat. No. 4.748,982 (Horzewski et al.) wherein a short sleeve or inner lumen at least about 10 cm in length is provided within the distal section of the catheter body which extends from a first port proximal to the balloon to a second port in the distal end of the guide wire and which is adapted to slidably receive a guide wire. The proximal port is not less than about 10 cm and not more than about 40 cm from the distal end of the catheter. Preferably, a slit is provided in the catheter body extending from the proximal port to a location proximal to the proximal end of the balloon to facilitate the rapid removal of the catheter from the proximal end of the guide wire which extends out of the patient.

Another improvement, which was introduced into the market place by Advanced Cardiovascular Systems, Inc., has been perfusion-type dilatation catheters which allow for long term dilatations to repair arterial dissections and other arterial damage. These perfusion catheters have a plurality of perfusion ports, in the wall forming at least part of the catheter body proximal to the balloon, which are in fluid communication with an inner lumen extending to the distal end of the catheter body. A plurality of perfusion ports preferably are provided in the catheter body distal to the balloon which also are in fluid communication with the inner lumen extending to the distal end of the catheter body. When the balloon on the distal extremity of the dilatation catheter is inflated to dilate a stenosis, oxygenated blood in the artery or other vessel (depending upon the location of the dilatation catheter within the coronary anatomy) passes through the proximal perfusion ports, through the inner lumen of the catheter body and out of the distal perfusion ports. This provides oxygenated blood downstream from the inflated balloon to thereby prevent or minimize ischemic conditions in the tissue distal to the catheter to thereby facilitate long-term dilatations. As is appreciated by those skilled in the art, tissue distal to a stenosis frequently is in jeopardy even before a dilatation procedure is commenced, due to pre-existing ischemic conditions. As a result, care should be exercised in sizing the perfusion ports and the inner lumen to ensure that there is adequate flow of oxygenated blood to the tissue distal of to the catheter, to eliminate or minimize ischemic conditions. Unfortunately, commercially available perfusion catheters have relatively large profiles, owing to the size of the inner tubular member which extends through the interior of the balloon. This prevents use of such catheters in many distal coronary locations with small diameter lumens.

A major and continual thrust of development work in the field of intravascular catheters. and particularly as to coronary angioplasty catheters, has been to reduce the profile, i.e., the transverse dimensions, of such catheters and to improve the flexibility thereof without detrimentally affecting the "pushability" characteristic of the catheters, particularly in the distal portion of the devices. A reduction in profile with little or no loss in pushability allows a dilatation catheter to be advanced much further into a patient's coronary vasculature and to cross much tighter lesions.

The guide wires employed in coronary angioplasty are of relatively small diameter because of the relatively small size of both the blood vessels and the luminal openings of the dilatation catheters which pass over the guide wires. To facilitate steering or placement within the cardiovascular system, a guide wire should be both relatively flexible toward its distal end and relatively rigid from a torsional standpoint over its entire length (i.e., it should posses "pushability" and "torqueability"). These two desirable properties are somewhat inconsistent with one another and therefore can be difficult to achieve in practice.

While guide wires designed for coronary angioplasty, at least in theory, also can be employed in the peripheral organs such as the arms and the legs, these guide wires may not have sufficient torsional rigidity for use in such applications. In addition, it is difficult to track a catheter with a relatively large luminal opening over a guide wire of relatively small diameter to a desired location. Ideally, the guide wire should fit closely within the luminal opening but still loosely enough to permit the catheter to move freely along the wire.

Further details of guiding catheters, dilatation catheters, guide wires, and other devices for angioplasty and other procedures can be found in U.S. Patent No. 4,323,071 (Simpson-Robert); U.S. Patent No. 4,439,185 (Lindquist); U.S. Patent No. 4,468,224 (Enzmann et al.): U.S. Patent No. 4,516,972 (Samson); U.S. Patent No. 4,438,622 (Samson et al.): U.S. Patent No. 4.554.929 (Samson et al.): U.S. Patent No. 4,582,185 (Samson); U.S. Patent No. 4,616,652 (Simpson): U.S. Patent No. 4,638,805 (Powell); U.S. Patent No. 4,748,986 (Morrison et al.); U.S. Patent No. 4,898,577 (Badger et al.): and U.S. Patent No. 4,827,943 (Taylor et al.).

A common problem that sometimes occurs after an angioplasty procedure has been performed is the development of restenosis at or near the original site of the stenosis. When restenosis occurs, a second angioplasty procedure or even bypass surgery may be required, depending upon the degree of restenosis. Currently, it is estimated that approximately one third of the patients who undergo PTCA procedures develop restenosis within six months. In order to prevent this occurrence and thus obviate the need to perform bypass surgery or subsequent angioplasty procedures, various devices and procedures have been developed for reducing the likelihood of development of restenosis after an arterial intervention. For example, an expandable tube (commonly referred to as a "stent"), designed for long-term implantation within the body lumen, has been used to help prevent restenosis. By way of example, several stent devices and stent delivery systems can be found in commonly assigned and commonly owned U.S. Patent No. 5,158,548 (Lau et al.); U.S. Patent No. 5,242,399 (Lau et al.); U.S. Patent No. 5,344,426 (Lau et al.): U.S. Patent No. 5,421,955 (Lau et al.); U.S. Patent No. 5,514.154 (Lau et al.); U.S. Patent No. 5,569,295 (Lam); and U.S. Patent No. 5,360,401 (Turnlund et al.).

More recent devices and procedures for preventing restenosis after arterial intervention employ a radiation source to minimize or destroy proliferating cells. Such proliferating cells are thought to be a major factor in restenosis development. Balloon catheters have been suggested as a means to deliver and to maintain the radiation source in the area at which arterial intervention has taken place, thereby exposing the area to a sufficient radiation dose to abate cell proliferation. Two such devices and methods are described in U.S. Patent No. 5,302,168 (Hess) and U.S. Patent No. 5,503,613 (Weinberger). Other devices and methods which utilize radiation treatment delivered by an intravascular catheter are disclosed in commonly assigned and commonly owned copending U.S. Serial No. 08/654,698, filed May 29, 1996. entitled Radiation-Emitting Flow-Through Temporary Stent.

Another medical device for the treatment of a body lumen by radiation is disclosed in European Patent Application No. 0 688 580 A1 (Schneider). In the Schneider device, the balloon catheter includes a lumen that extends from a proximal opening to an area near the distal end of the catheter, where it dead ends. This lumen. known as a "blind" or "dead end" lumen, is intended to carry a radioactive-tipped source wire that s!ides into the lumen, once the catheter is in place in the artery or body lumen. When the source wire is positioned, the radioactive section at the distal tip lies near the dead end to provide radiation to the body lumen.

Another procedure used to deliver a radiation source to a vessel is disclosed in U.S. Patent No. 5,503,613 (Weinberger), wherein a catheter is provided with two inner lumens. One lumen accepts a guide wire for sliding into the body lumen. The other lumen is a blind lumen and receives a radiation dose delivery wire manipulated remotely by a computer controlled afterloader. After the catheter has been positioned so that its distal end lies just distally of the stenosed area, the radiation dose delivery wire is inserted into the open end of the blind lumen and advanced to the dead end, where it delivers a radiation dose to the affected tissue. This method requires using a catheter with a rather large cross-section, i.e., a cross-section that is sufficient to accommodate both lumens. which can complicate the insertion of the catheter in narrow, tortuous arteries. Another method avoids this problem by using an over-the-wire catheter to treat the stenosed region. and then withdrawing the guide wire and inserting the radiation dose delivery wire in its place. Unfortunately, with this latter device, the radiation source wire will be exposed to the blood, requiring sterilization if reuse is contemplated, or disposal after one use, which increases the cost associated with the procedure and further can increase the quantity of radioactive waste as to which stringent disposal requirements apply.

The use of nuclear radiation to prevent restenosis represents a significant improvement in the safety and success rate of PTCA and percutaneous transluminal angioplasty (PTA) procedures. However, a few attendant factors give rise to special considerations that must be addressed to successfully employ this method. In particular, withdrawing the guide wire is disfavored by physicians because it results in a more complex and lengthy procedure, thereby increasing the risk of complications. Further, if the radiation source delivery wire comes in direct contact with the patient's blood, it poses the risk of blood contamination. Thus such a direct contact wire must be sterilized before it is retracted into the afterloader and used on another patient. In addition, the salts and other chemical compounds found in blood may adversely impact the radiation source delivery wire and shorten its useful life.

Another consideration uniquely implicated in intraluminal radiation therapy is that the radiation source must be located centrally within the body lumen being treated to assure uniform delivery of the radiation dose to the entire target area. Typically, the radioactive sources employed are gamma ray emitters, and point-source gamma rays attenuate inversely with the square of the distance traveled. If the radiation dose delivery wire lies closer to one side of the lumen than to another or at an angle to the lumen, the radiation dose delivered will be non-uniform along the entire length of the target area and some areas thus likely will receive appreciably larger doses than others. Precise spatial alignment of the radiation dose delivery wire is difficult to achieve in practice. Although this problem is not encountered when the radiation source is embedded in the tip of the catheter, which tip usually becomes centered within the lumen when the balloon is inflated, the use of catheters to deliver the radiation dose can result in excessive radiation doses being delivered to the body lumens which lead to the stenosed area because the catheter typically must remain in place in the lumen longer than other means of site-specific radiation delivery. Inserting and withdrawing catheters repeatedly can cause additional damage to the vascular tissue owing to the relatively large cross-section of the catheters.

Despite many advances in the field, there remains a need for an apparatus that will minimize the time required to accurately deliver a radiation source to a body lumen following an intravascular intervention such as PTCA and that will prevent or minimize the exposure of the radioactive source material to the blood stream. Such a device ideally will combine the best features of both catheters and radiation source delivery wires into one novel and effective package that further will enhance the safety and success rate of intravascular procedures.

### SUMMARY OF THE INVENTION

Certain preferred embodiments of the present invention provide an apparatus and method of treating a body lumen, such as a coronary artery or other artery or vein, with radiation to reduce the likelihood of the development of restenosis. In these embodiments, a guide wire is provided which is adapted to receive a radiation source for delivery to a target area in the body lumen. Typically, the radiation source will be delivered through a lumen in the guide wire to irradiate the target area. The term "target area" as used herein means that portion of a body lumen which is to receive a radiation dose. In one embodiment there is provided a hollow guide wire having proximal and distal ends, a radiation source lumen extending from the proximal end to a point proximate the distal end, and a solid core portion at the distal end. A flexible tip may be secured to the solid core portion to facilitate inserting and navigating the guide wire through tortuous body lumens. The radiation source lumen is adapted to concentrically and slidably receive a radiation source delivery wire, and the solid core portion defines the maximum distance the radiation source wire can be inserted into the radiation source lumen. Radiopaque markers may be provided on the outer surface of the hollow guide wire to assist in the precise positioning of the solid core portion and to insure accurate positioning of the radiation source wire relative to the target area.

In another embodiment there is provided an assembly for delivering the radiation source within a body lumen to the target area. The hollow guide wire having the radiation source lumen extending from the proximal end of the guide wire through to a point proximal to the distal end has a solid core portion proximate the distal end. A catheter is provided having a guide wire lumen which extends through at least a portion of the guide wire, the guide wire lumen being sized for receiving the guide wire. The guide wire lumen will extend through the catheter for over-the-wire applications. and will extend from a side port on the outer surface of the catheter through the distal end in rapid-exchange catheter applications. An expandable member. such as a dilatation balloon, is associated with the distal end of the catheter for centering the guide wire within the body lumen, such as a coronary artery. A radiation source, such as a radiation source wire, is received in the radiation source lumen, and the radioactive portion of the radiation source wire is positioned proximate the distal end of the guide wire with the solid core portion of the guide wire defining the distal-most extent to which the radiation source can be advanced. The radiation source should be positioned within the radiation source lumen concentric with the balloon portion of the catheter. which is positioned at the target area, to deliver the radiation dose.

The method for irradiating the target area includes the assembly described for delivering a radiation dose within the body lumen. The guide wire is advanced within the body lumen such that the tip of the guide wire is advanced distally of the target area. The catheter is advanced over the guide wire so that the expandable member or balloon is positioned within the target area, so that the balloon portion of the catheter can be inflated into contact with the target area, thereby centering the guide wire and radiation source lumen within the target area. The radiation source, such as a radiation source wire, is advanced within the radiation source lumen of the guide wire so that a radioactive isotope can be positioned within the target area, to deliver a radiation dose thereto. After treatment, the radiation source is withdrawn from the radiation source lumen. the balloon portion of the catheter is deflated and the catheter is withdrawn from the body lumen as well as the guide wire.

In either an over-the-wire or rapid-exchange catheter application. it is contemplated that perfusion holes proximal and distal to the balloon portion of the catheters will be provided to perfuse blood during the irradiating process.

The radiation therapy can be provided either during a PTCA or PTA procedure, or immediately thereafter. While it is contemplated that the target area can be irradiated prior to a dilatation procedure, such is not currently the preferred sequence. Preferably, after a dilatation procedure such as a PTCA or PTA procedure is completed, and without withdrawing either the dilatation catheter or the guide wire. the irradiation procedure is accomplished as described.

Other features and advantages of the invention will become apparent from the following detailed description, taken in conjunction with the accompanying drawings, which illustrate, by way of example, the features of the invention.

### BRIEF DESCRIPTION OF THE DRAWINGS

FIGURE 1 is a longitudinal cross-sectional view of a tubular guide wire and a position of the radiation source delivery wire embodying features of the present invention.

FIG. 2 is an elevational view, partially in section, illustrating a rapid-exchange catheter receiving the tubular guide wire shown in FIG. 1.

FIG. 3 is an elevational view, partially in section, illustrating an over-the-wire catheter receiving the tubular guide wire and the radiation source wire inserted within the guide wire.

FIG. 4 is an elevational view, partially in section. illustrating the rapid-exchange catheter and guide wire of FIG. 2, where the guide wire has received the radiation source wire.

FIG. 5 is an enlarged cross-sectional view of the distal end of the catheter, illustrating the guide wire and the radiation source wire positioned at the target area to irradiate the area.

FIG. 6 is a cross-sectional view of a guide wire embodying the invention in which at least a portion of the distal end of the guide wire is formed of a radiation transparent material such as a polymer.

### DETAILED DESCRIPTION OF THE PREFERRED EMBODIMENTS

Intravascular procedures such as percutaneous transluminal coronary angioplasty (PTCA) or percutaneous transluminal angioplasty (PTA) sometimes result in development of restenosis in the treated or target area. A promising new technology that has been used successfully to prevent development of restenosis delivers a pre-selected dose of nuclear radiation to the target area during or immediately following the angioplasty procedure. The radiation dose usually is delivered via a wire which incorporates a radioactive source material. The wire is inserted into the catheter used to treat the stenosed area. This procedure requires that the guide wire first be withdrawn from the catheter to permit insertion of the radiation dose delivery wire in the guide wire lumen in the catheter, or that the catheter incorporate an additional lumen that receives the radiation dose delivery wire. The latter dual-lumen catheters, however, have higher profiles which are undesirable.

Certain preferred embodiments of the present invention obviate the need for a dual-lumen catheter and also makes it unnecessary to remove the guide wire prior to radiation treatment by providing, generally, a tubular guide wire 25 formed with a radiation source lumen 26 extending through a portion of the guide wire. The guide wire 25 has an opening at a proximal end 27 thereof for slidably receiving a radiation source wire 50, and formed at distal end 29 of the guide wire 25 is a flexible tip 31 useful in navigating tortuous vessels.

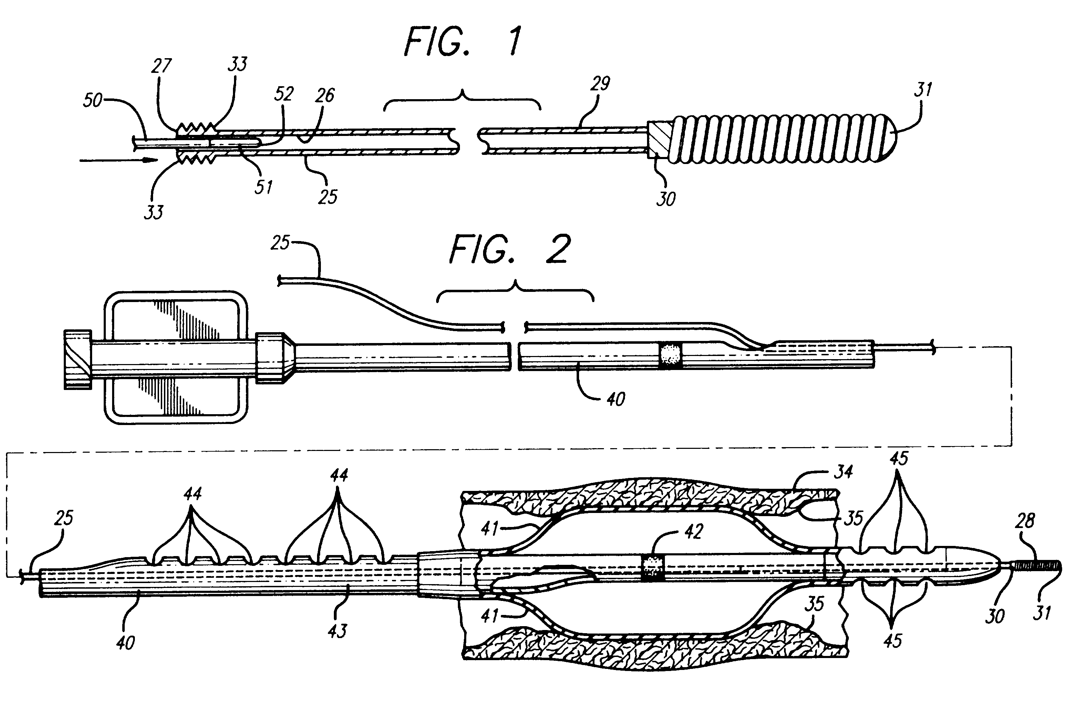

Referring to FIG. 1, the guide wire 25 may be formed from any high tensile strength material such as stainless steel, titanium, or a suitable superelastic nickel-titanium (NiTi) alloy, and may be formed by methods such as multiple extrusion of a tube through repeatedly smaller dies until the desired dimensions are achieved. It is important that the guide wire thus formed provide the critical characteristics of "pushability" and "torqueability" which allow it to be pushed along the tortuous paths defined by the body lumens into which it is introduced.

If the guide wire is formed from NiTi, the preferred superelastic alloy has a final austenite transformation temperature (A_{f}) of less than about 40°C and contains about 32 percent to about 52 percent titanium with the balance of the composition being nickel, and up to 10 percent of additional alloying elements. A nickel content in excess of 50 percent causes brittleness and prevents effective cold working. The additional alloying elements may include iron, cobalt, chromium, platinum, palladium, tungsten, vanadium, copper and beryllium.

Alternatively, the guide wire 25 may be constructed of polymer or composite distal portion which is secured to a metal alloy proximal portion by any suitable method such as by welding, brazing. or the use of epoxies. Such construction would provide the desirable "pushability" and "torqueability" characteristics of metal over the majority of the length of the guide wire, but would offer the flexibility inherent with polymers at the distal extremity of the guide wire, thereby facilitating the insertion and positioning of the guide wire within the patient's vascular system.

A suitable outer diameter for the guide wire 25 is in the range of 0.203 to 0.813 mm (0.008 to 0.032 in.) and preferably of approximately 0.356 mm (0.014 in.). A guide wire with a 0.356 mm (0.014 in.) outer diameter is compatible with most dilatation catheters presently on the market. The guide wire 25 shown in FIG. 1 has a constant outer diameter along its entire length, but it will be understood by those skilled in the art that the guide wire 25 also may be formed so as to exhibit a tapered configuration which has a larger outer diameter at the proximal end 27 and a smaller diameter at the distal end 29. The overall length of the guide wire 25 can be in the range of 50 to 250 cm, and for coronary applications preferably is approximately 175 cm long. The guide wire also may be provided with a radiation shielding means if the radioactive source material to be used so warrants. The radiation shield may be comprised of a flexible metal covering (not shown) which encases the guide wire 25 to a point near the solid distal end, thereby defining a short window through which radiation can emit forth into the surrounding tissue. Such a metal covering may be of tubular construction or, alternatively, may comprise of a coating around the guide wire.

Alternatively, a guide wire constructed of two materials, as will be described herein, could obviate the need for a separate radiation shield, and the metal alloy portion of the guide wire could be formed of sufficient thickness to act as a radiation shield while the distal polymer portion would provide a window through which the emitted radiation can penetrate.

The inner diameter of the guide wire 25, namely, the radiation source lumen 26, will vary depending upon the intended application and the degree of flexibility required, as well as on the outer diameter of the guide wire 25. It is well known that the section modulus of a tube, a measure of the capacity of a section of the tube to resist any bending moment applied to it, decreases with the thickness of the tube wall. A guide wire inner lumen diameter to outer diameter ratio of 0.32, for example, will result in a decrease in the section modulus of approximately one percent as compared to a solid tube. for example of the type used for conventional guide wires for use in PTCA and PTA procedures. The section modulus of a tube having a solid core varies with the cube of its diameter, and for this reason a larger diameter tubular guide wire can be formed with a larger lumen and yet provide the same section modulus as a smaller guide wire having a smaller lumen. In a preferred embodiment, the guide wire 25 has an outer diameter of 0.356 mm (0.014 in.) and the radiation source lumen 26 has a diameter that preferably is in the range of about 0.254 to 0.114 cm (010 to 0.0045 in.)

The guide wire has a solid core portion 30 at the distal end 29 of the guide wire, which may be formed contiguous with the hollow portion (i.e., radiation source lumen 26) of the guide wire during the extrusion process, or which may be attached to the hollow portion by any secure means such as by welding, brazing, or the use of an epoxy. The method used should achieve a bond with a section modulus substantially equal to the section modulus of the guide wire hollow portion in order to prevent separation of the solid core portion 30 while the guide wire is in the patient. The solid core portion includes a flexible tip 31, to aid navigating the guide wire through tortuous body lumens. The flexible tip 31 may be attached to the solid portion by any suitable method or may be formed from the same core element such as by centerless grinding. One or more radiopaque markers 42 may be provided on the guide wire 25 to allow the physician to see the position of the guide wire distal end 29 on a fluoroscope. Alternatively, a section of the solid core portion 30 may be formed from a radiopaque material such as gold.

The proximal end 27 of the guide wire 25 may be provided with a fastening mechanism 33, such as a threaded fastener or similar mechanism for connecting the guide wire 25 to a computer controlled afterloader (not shown). The fastening mechanism 33 preferably will be both air-tight and fluid-tight, and therefore will be suited for use with any of solid, liquid or gaseous radioactive sources. The fastening mechanism 33 also should be rotatably mounted to the guide wire, so that the circular position of the guide wire can be adjusted as necessary, even after the guide wire has been connected to the afterloader. Alternatively, the fastening mechanism may be spring-loaded. or a combination threaded/spring loaded assembly.

In operation. the guide wire 25 is inserted into the patient's vascular system much in the same manner that conventional guide wires are inserted when used in PTCA or PTA procedures. The flexible tip 31 at the distal end 29 can be shaped manually by the physician into a desired configuration prior to insertion into the body lumen. The specially-shaped flexible tip 31 facilitates inserting and navigating of the guide wire 25 through the body lumen, and the radiopaque markers 42 allow the physician to track the location of the guide wire on a fluoroscope.

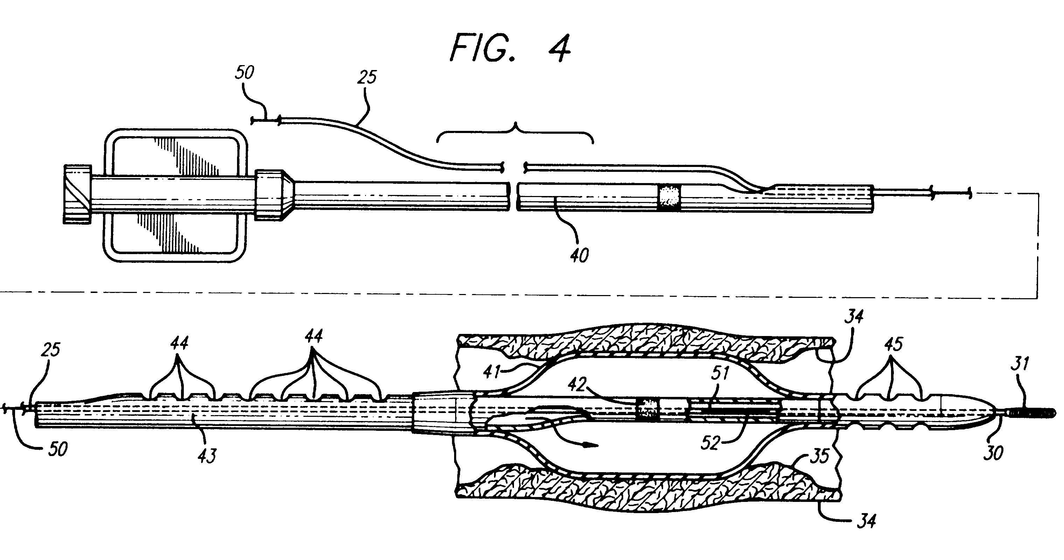

In conjunction with the guide wire, the preferred method of use is described. Referring to FIGS. 2, and 4-5, the guide wire 25 is advanced in an artery 34 until the distal end 29 is located just past the stenosed area, as is known in the art. The dilatation catheter 40 is advanced distally over the guide wire 25 until the inflatable balloon 41 of the catheter is positioned across the stenosed or target area 35. A radiopaque marker 42 usually is provided on the catheter at the midpoint of the inflatable balloon to aid the physician in tracking the progress of the catheter within the body lumen and in positioning the balloon within the target area 35. The dilatation catheter 40 includes a guide wire lumen 43, which is sized to slidably receive the guide wire 25 and to maintain it in a coaxial relationship with the catheter. The guide wire lumen 43 preferably is 0.0254 to 0.051 mm (0.001 to 0.002 in.) larger than the outer diameter of the guide wire. Thus, in a preferred embodiment, the guide wire 25 has an outer diameter of 36 mm (.014 in.), and the guide wire lumen 43 preferably has an inner diameter of about 0.381 to 0.406 mm (.015 to .016 in.)

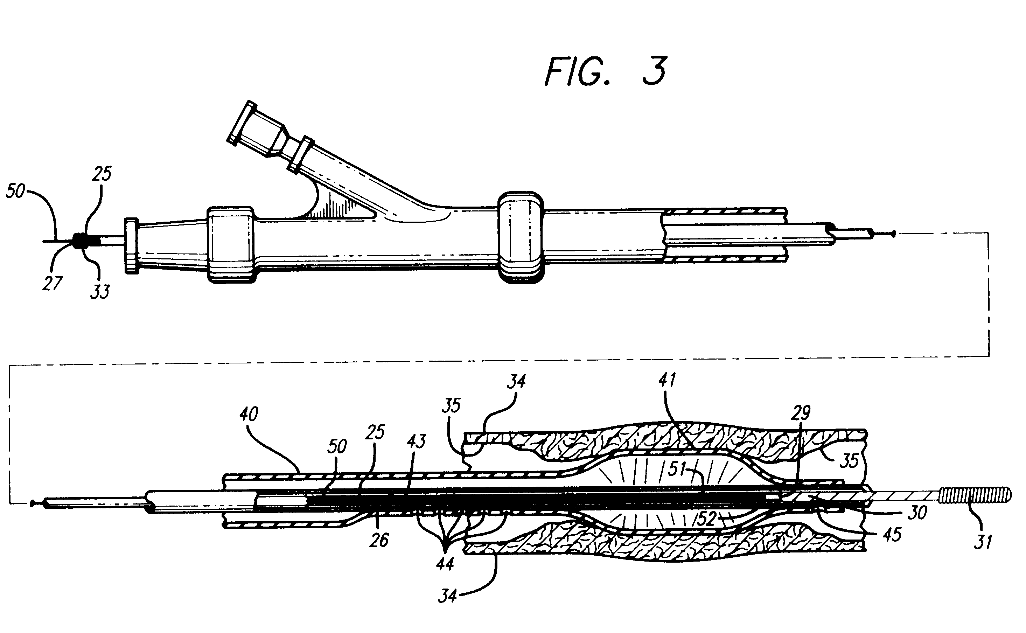

The dilatation catheter 40 next is used to treat the stenosis by inflating the balloon 41 with a radiopaque liquid for a selected period of time. The catheter 40, shown in FIG. 2, is a rapid-exchange catheter generally known in the art and has a plurality of perfusion ports 44, 45. It will be understood by those skilled in the art, however, that the guide wire works equally well with all other types of catheters, including over-the-wire-type catheters. Thus, as is shown in FIG. 3, the guide wire is used with an over-the-wire catheter 40 also having the capacity for perfusion. (Similar or like devices herein are referred to with the same reference numerals in the drawing figures.) The guide wire is uniquely well adapted for use with perfusion-type catheters. because the guide wire allows the radioactive source to be inserted into the patient without exposure to the patient's blood stream. thereby eliminating the risk of contaminating the blood stream or the wire. In extreme cases. exposing the radiation source material to the blood of the patient may contaminate the blood and cause further complications for the patient. Therefore, it is highly desirable to isolate the blood stream from the radiation source while allowing blood flow through the body lumen being treated, two previously incompatible goals which is now achievable by the described embodiment.

Referring to FIGS. 1-5, following dilation of the stenosed area 35, a radiation source, such as a wire 50 having a radioactive source material 51 formed in a distal end 52 thereof, is inserted into the proximal end 27 of the radiation source lumen 26 and is advanced through the lumen, adjacent to the solid core portion 30 of the guide wire 25. The solid core 30 limits the extent to which the radiation source wire 50 can be advanced distally into the guide wire 25 through the radiation source lumen 26, and because the solid core portion 30 previously has been precisely located by the physician within the patient's artery by monitoring the location of the radiopaque markers 42 on a fluoroscope. the physician now can determine with certainty the location of the radioactive source material 51 within the patient's body lumen.

The inflated balloon 44 pushes evenly against the walls of the artery and thus serves to center the catheter 40 within the artery 34. and thereby also centers the guide wire 25, and the radioactive source material 51 within it. If the radiation source is in the form of a wire 50, it should be of a suitable diameter to be slidably received and coaxially situated in the radiation source lumen 26, and preferably is about 0.0254 to 0.051 mm (0.001 to 0.002 in.) smaller than the diameter of radiation source lumen 26. This is an important consideration in order to ensure that the radioactive source material 51 is centered within the body lumen so that the entire target area 35 receives an equal dose of radiation.

In oncological procedures. radiation wires typically are inserted and advanced in the patient by a computer-controlled afterloader. and the guide wire of the present invention is especially well suited for use with an afterloader. The proximal end 27 of the guide wire 25, for instance. may be formed with a fastener 33 adapted for connection to the afterloader (not shown), thereby allowing completely hands-free insertion of the radiation source wire 50. Using an afterloader also would allow precise positioning of the radiation source wire 50, because the guide wire 25 is of a known length and the afterloader computer thus can calculate the precise distance to which the radiation source wire must be advanced in order to position the radioactive source material 51 at the target area 35.

The hollow guide wire 25 allows delivery of the radiation dose immediately following or, indeed, even during the PTCA or PTA procedure. Conventional guide wires typically must be removed to make room to insert a radiation source wire, or alternatively an additional lumen must be provided in the catheter to receive the radiation source wire, which additional lumen would increase the catheter profile. The task of exchanging wires is time consuming and may cause further complications, especially if a perfusion-type catheter is not being used and normal blood flow through the patient's arteries is compromised for a long period, thus depriving the tissue distal of the catheter of an adequately oxygenated blood supply, resulting in ischemia. Further. inserting and removing guide wires repeatedly increases the risk of traumatically engaging a vessel wall and, when a perfusion-type catheter is used, the risk of inadvertently passing one of the wires through a perfusion port also is increased. Therefore, it is desirable to minimize the movement of the guide wire after it properly has been positioned at the target area, as can be achieved through use of the guide wire of the present invention. A system like the one described having a single lumen to carry both the guide wire and the radiation source material also is beneficial insofar as the expense and complications associated with manufacturing a multiple lumen catheter are avoided. From a procedural perspective. such a single lumen construction also is advantageous as compared to a multi-lumen construct because generally speaking, the greater is the number of catheter lumens, the greater is the overall catheter outer diameter, and the greater the outer diameter. the greater is the profile and the stiffness of the system. The stiffer the system. the greater the resistance that is encountered when trying to negotiate the circuitous path to the target area through the tortuous vascular anatomy. The greater the resistance, the more likely it is that a part of the vasculature will be damaged during the procedure.

In one preferred method of use, the radiation source wire 50 also may be positioned within the guide wire 25 concurrently with the inflation of the catheter balloon 41, thereby greatly reducing the total time required for performing the dilatation procedure. In this method, the catheter 40 and the guide wire 25 are advanced and positioned in the artery 34 in the conventional manner for a dilatation procedure. The proximal end 27 of the guide wire 25 is connected to an afterloader using the fastener 33 or a similar connector. The radiation source wire 50 is advanced by the computer-controlled afterloader into the radiation source lumen 26 of the guide wire 25. Because the distance from the proximal end 27 of the guide wire to the center of the balloon 41 is known, the radiation source wire 50 is advanced until the radioactive source material 51 is positioned within the balloon 41. At the same time the balloon is expanded to enlarge the stenosed region 35, the radioactive source material 51 will be emitting radiation at a predetermined rate and dose level. The guide wire remains in position for the entire procedure. which greatly reduces the overall time of the procedure and avoids the complications associated with the prior art devices. The radiation source wire 50 can be withdrawn into the afterloader when the predetermined dose level has been administered. which may be during or immediately after the dilatation procedure. It is contemplated that the guide wire can be used with the radiation source wire to irradiate the target area before the dilatation procedure, however, there is little data at present that would suggest that such a procedure would be beneficial.

The guide wire also can be employed solely as a radioactive dose delivery means in situations that do not require the insertion of a catheter. In such situations, the guide wire 25 would act as a catheter, but with a much lower risk of damaging the body lumens and with much enhanced ease of inserting and navigating within the lumens.

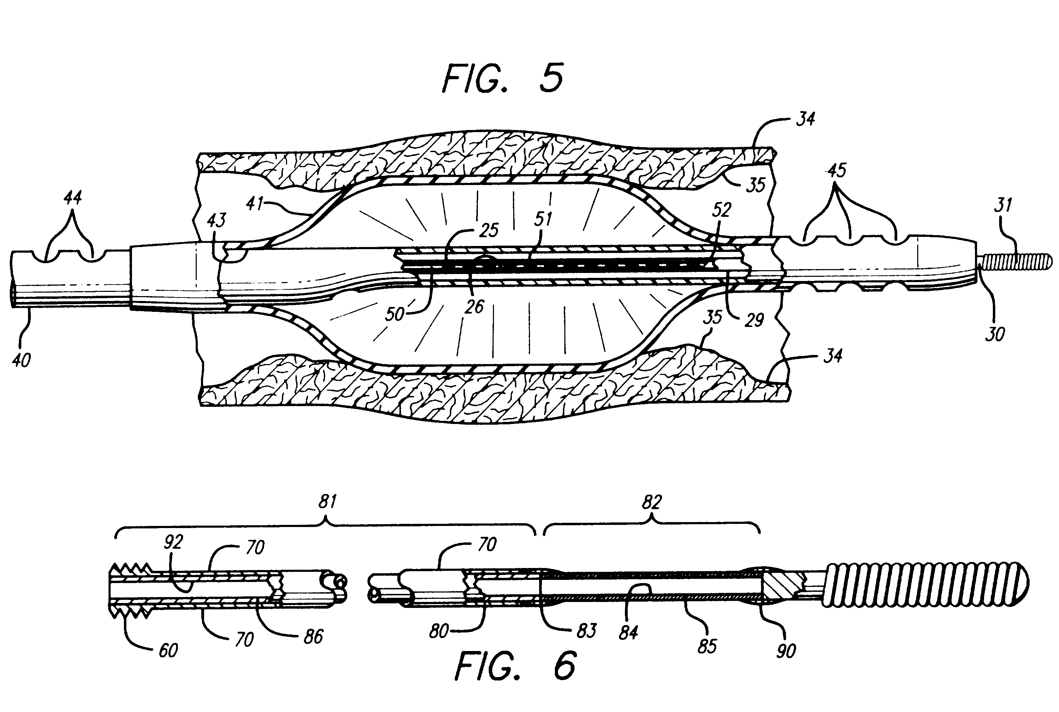

Referring to FIGS. 1 and 3-5, the radiation source has been illustrated as being in the form of a wire 50. It will be understood by those skilled in the art, however, that the guide wire 25 is equally well suited for use with radioactive source materials that are gases. liquids, or slurries of solid particles suspended in solution. This is possible due to the monolithic construction of the guide wire and the provision of the containing lumen 26. which minimizes the risk of leaks developing such that the radioactive source materials can leak out or the blood can leak in to become contaminated. A guide wire thus can be fastened in a leak-proof manner to an afterloader and the afterloader then can evacuate the radiation source lumen 26 and discharge a radioactive liquid or gas into the guide wire 25 for a predetermined period of time. This method could necessitate the use of radiation shield 70 (FIG. 6) around the guide wire 25, to prevent irradiating healthy tissue or personnel in the catheterization laboratory ("cath lab").

Referring to FIG. 6, an alternative embodiment of the invention is shown in which a guide wire 80 is formed of a radiopaque metal alloy at a proximal end section 81, while distal end section 82 is formed of a polymer that is substantially transparent to radiation. The distal end section 82 can be attached to the proximal end section 81 by any suitable known means such as by adhesive bonding, laser welding, or the like. An annular bond 83 at the juncture of the two guide wire sections 81, 82 should ensure a secure connection. Likewise, the distal end section 82 is securely attached to a solid core portion 84 of the guide wire 80 by an annular bond 90. As previously was described for other embodiments of the invention, the guide wire 80 includes a radiation source lumen 92 having an inner diameter that is adapted to receive a radiation source such as the radiation source wire 50. The distal end section 82, being formed from a radiation transparent polymer, will permit maximum radiation exposure to the target area 35, when the radiation source material 51 is positioned within the distal end section 82. The proximal end section 81 is formed from, for example, a stainless steel alloy or another conventional guide wire metal alloy which is radiopaque, so that as the radiation source material 51 is advanced through the guide wire 80, it will not penetrate the metal alloy of the proximal end section 81. The proximal end section 81 thus protects the patient and the medical personnel from radiation exposure, while the distal end section 82 selectively permits the radiation source material to emit radiation to the target area 35. As described, the radiation shield 70, in the form of a covering or jacket, can be applied to cover the proximal end section 81 to further insure that no radiation, or that inconsequential amounts of radiation. penetrate the proximal end section. The distal end section 82 should have a length that is consistent with the length of the inflatable balloon of the catheter, typically 20 mm to 40 mm for most coronary applications, but the length can vary depending on the particular application.

While there are numerous radioactive sources available for use with the described embodiments the following radioisotopes are preferred: Iridium 192; Sodium 22; Scandium 46; Manganese 54; Yttrium 88; Cerium 139; Cerium 141; Strontium 85; Cobalt 57; Cobalt 60; Cesium 134; Palladium 103; Gold 198; Niobium 95; Mercury 203; Iodine 125; and Iodine 131. While gamma radiation is preferred, beta radiation also is contemplated, although it may not penetrate as well as gamma radiation through the guide wire.

From the foregoing, it will be appreciated that embodiments of the present invention provide a guide wire for use in intravascular procedures that allows delivery of a radiation source without the use of a high profile, dual-lumen catheter and without exposing the radiation source to the blood stream, the embodiments further greatly reduce the time required for performing all intravascular procedures which incorporate radiation treatment of the affected area. Those skilled in the art will recognize that the invention is suitable for all types of catheters used with guide wires and may be used in a variety of body lumens. The present invention further advances the state of the art for intravascular procedures by offering versatility in the types of radioactive source materials that can be used, flexibility in the range of possible applications, and safety to the patient and attending medical staff.

While particular embodiments of the invention have been illustrated and described, various modifications can be made without departing from the spirit of the invention, and all such modifications and equivalents are intended to be covered.

## Claims

1. An apparatus for positioning a radiation source in a body lumen, comprising:
a guide wire (25. 80) having a proximal end (27), a distal end (29), and a radiation source lumen (26, 92) extending from the proximal end through the guide wire to a point near the distal end;
a solid core portion (30, 84 at the distal end (29) of the guide wire (25, 80);
a flexible guide wire tip (31) at the distal end (29) of the guide wire (25, 80) for navigating through the body lumen;
the radiation source lumen (26, 92) adapted to receive a radiation source (51) to be positioned proximate the distal end (29) of the guide wire (25, 80) with the solid core portion (30) defining the distal-most extent to which the radiation source (51) is advanced into the lumen.

2. The apparatus of claim 1, wherein the guide wire (25, 80) has radiopaque markers (42) formed thereon for viewing on a fluoroscope.

3. An assembly for positioning a radiation dose within a body lumen, comprising:
a guide wire (25, 80) having a proximal end (27), a distal end (29), and a radiation source lumen (26, 92) extending from the proximal end through the guide wire to a point proximate the distal end;
a solid core portion (30, 84) proximate the distal end of the guide wire;
a guide wire tip (31) on the distal end (29) of the guide wire for navigating through the body lumen;
a catheter (40) having a guide wire lumen (43) extending through at least a portion thereof, the guide wire lumen sized for receiving the guide wire (25, 80);
a radiation source material (51) received in the radiation source lumen (26, 92) and positioned proximate the distal end (29) of the guide wire (25, 80) with the solid core portion (30, 84) defining the most distal extent to which the radiation source material (51) can be inserted into the radiation source lumen; and
a balloon (41) concentric about the catheter (40) and inflatable to contact the walls of the body lumen (34) and to center the catheter (40) and the radiation source material (51) within the body lumen.

4. The assembly of claim 3, wherein the catheter guide wire lumen (43) and the guide wire (25, 80) are sized such that the guide wire is received concentrically within the catheter guide wire lumen.

5. The assembly of claim 4, wherein the guide wire (25, 80) has an outer diameter of approximately 0.0254 to 0.051 mm (0.001 to 0.002 in. ) smaller than an inner diameter of the catheter guide wire lumen (43).

6. The assembly of claim 3, wherein the catheter (40) is selected from the group comprising a rapid-exchange-type catheter; an over-the-wire-type catheter; and a perfusion-type catheter.

7. The invention of claim 1 or claim 3, wherein the guide wire (25,80) is tapered, the guide wire proximal end (27) having a larger outer diameter than the outer diameter of the guide wire distal end (29).

8. The invention of claim 1 or claim 3, wherein the guide wire (25,80) further comprises means for shielding (70) the body lumen (34) from the radiation emitted by the radiation source (51).

9. The invention of claim 8, wherein the radiation shielding means (70) includes a metallic cover disposed around a portion of the guide wire (25,80).

10. The assembly of claim 3, wherein the catheter (40) further comprises means for shielding (70) the body lumen (34) from the radiation emitted by the radiation source material (51).

11. The assembly of claim 10, wherein the radiation shielding means (70) includes a metallic cover disposed around at least a portion of the catheter (40).

12. The invention of claim 1 or claim 3, wherein the guide wire (25,80) has an outer diameter substantially in the range of 0.203 to 0.813 (0.008 to 0.032 in.).

13. The invention of claim 12, wherein the radiation source lumen (26,92) has an inner diameter substantially in the range of 0.114 to 0.254 (0.0045 to 0.010 in.).

14. The invention of claim 13, wherein the ratio of the radiation source lumen (26,92) inner diameter to the guide wire (25,80) outer diameter is no greater than 0.32.

15. The invention of claim 1 or claim 3, wherein at least a portion (81) of the guide wire (25,80) is formed from a metal alloy selected from the group of metal alloys including stainless steel, nickel-titanium, titanium, tungsten, platinum, iron, cobalt, chromium, palladium and copper-beryllium.

16. The invention of claim 1 or claim 3, wherein at least a portion (82) of the guide wire (25,80) is formed from a flexible polymer material.

17. The invention of claim 1 or claim 3, wherein the guide wire (25,80) includes a distal section (82) formed from a polymer material attached to a proximal section (81) formed from a metal alloy selected from the group of metal alloys including stainless steel, neckel-titanium, titanium, tungsten, platinum, iron, cobalt, chromium, palladium and copper-beryllium,

18. The invention of claim 1 or claim 3, wherein the guide wire (25,80) is approximately 50 cm to 250 cm long.

19. The invention of claim 1 or claim 3, wherein the radiation source material (51) is formed from the group of radiation source materials including a radioactive solid material, a radioactive liquid, a radioactive gas and a radioactive slurry.

20. The invention of claim 19, wherein, when the radiation material (51) is formed from a solid material, the radiation source lumen is adapted to coaxially receive the radiation source material (51) associated with a radiation source wire (50).

21. The invention of claim 19, wherein, when the radiation material (51) is formed from a solid material, the radiation source lumen (26,92) is adapted to receive a radiation source wire (50) that is advanced within the radiation source lumen by a remote afterloader.

22. The invention of claim 21, wherein the guide wire distal end (29) is provided with a fastener (33) for connecting the guide wire to the afterloader.

23. The invention of any of claims 20 to 22, wherein the radiation source wire (50) has an outer diameter approximately 0.0254 to 0.051 (0.001 to 0.002 in.) smaller than the inner diameter of the radiation source lumen of the guide wire.

24. The invention of claim or claim 3, wherein the radiation source material (51) comprises radioisotopes selected from the group of radioisotopes including Iridium 192, Sodium 22, Scandium 46, Manganese 54, Yttrium 88, Cerium 139, Cerium 141, Strontium 85, Cobalt 57, Cobalt 60, Cesium 134, Palladium 103, Gold 198, Niobium 95, Mercury 203, Iodine 125 and Iodine 131.

25. A method for irradiating a portion of body lumen, the method comprising:
providing an assembly having a catheter (40) with an expandable member (41) at a distal end thereof and a guide wire lumen (43 ) extending through at least a portion thereof, the guide wire (25, 80) lumen sized for receiving a guide wire therein, the guide wire having a proximal end (27). a distal end (29). and a radiation source lumen (26, 92) extending from the proximal end through the guide wire to a point proximate the distal end, and a solid core portion (30, 84) on the distal end of the guide wire;
positioning the catheter and the guide wire in the body lumen (34);
advancing the guide wire (25, 80) so that the guide wire distal end is positioned distal to a target area (35) in the body lumen (34);
advancing the catheter (40) over the guide wire (25. 80) so that the expandable member (41) is positioned within the target area (35) and inflating the expandable member into contact with the body lumen (34);
advancing a radiation source material (51) within the radiation source lumen (26. 92) so that a radioactive isotope is positioned within the target area;
irradiating the target area (35) for a predetermined time and predetermined radiation dose level;
withdrawing the radiation source material (51) from the radiation source lumen (26, 92); and
deflating the expandable member (41) and withdrawing the catheter (40) and guide wire (25, 80) from the body lumen (34).

26. The method of claim 25, wherein the step of providing a catheter (40) includes any of a rapid-exchange catheter, an over-the-wire catheter, a fixed wire catheter, or a perfusion catheter.

27. The method of claim 25, wherein the step of advancing the radiation source material (51) includes advancing a radiation source wire (50) having a radiation source material associated with a distal end (52) of the wire so that, as the wire is advanced in the radiation source lumen, the radiation source material at the distal end of the wire will be positioned within the target area (35).

28. The method of claim 27, wherein the advancing step further comprises advancing the radiation source wire (50) using an afterloader and the withdrawing step further includes withdrawing the radiation source wire using the afterloader.
